(19) Europäisches Patentamt

European Patent Office

Office européen des brevets

(11) **EP 0 895 080 B1**

(12) **FASCICULE DE BREVET EUROPEEN**

(45) Date de publication et mention
de la délivrance du brevet:
**24.01.2001 Bulletin 2001/04**

(51) Int Cl.⁷: **G01N 33/28**

(21) Numéro de dépôt: **98401883.8**

(22) Date de dépôt: **24.07.1998**

(54) **Procédé et dispositif de décalaminage d'une chambre de combustion d'un moteur diesel**

Verfahren und Vorrichtung zur Entzunderung einer Verbrennungskammer in einem Dieselmotor

Method and apparatus for descaling a combustion chamber in a diesel engine

(84) Etats contractants désignés:
**DE ES FR GB IT**

(30) Priorité: **29.07.1997 FR 9709649**

(43) Date de publication de la demande:
**03.02.1999 Bulletin 1999/05**

(73) Titulaire: **ELF ANTAR FRANCE**
**92400 Courbevoie (FR)**

(72) Inventeurs:
• **Mille, Guy**
**69700 Echalas (FR)**

• **Cellier, Joseph**
**69200 Venissieux (FR)**

(74) Mandataire: **Timoney, Ian Charles Craig**
**Elf Exploration Production**
**Département Propriété Industrielle**
**Tour Elf**
**EP/T/RD/DPI - Bureau 34 G 47**
**92078 Paris La Défense Cedex (FR)**

(56) Documents cités:
**EP-A- 0 610 118**      **DE-B- 1 270 838**
**FR-A- 2 702 004**      **US-A- 3 469 954**

## Description

### DOMAINE TECHNIQUE

La présente invention concerne un procédé et un dispositif de décalaminage d'une chambre de combustion d'un moteur diesel, ce moteur étant destiné à la mesure de l'indice de cétane de carburants d'alimentation des moteurs diesels.

Elle trouve son application dans les laboratoires de recherche, les laboratoires de contrôle et les unités de fabrication de ces carburants dans les usines de traitement de pétrole brut.

### ETAT DE LA TECHNIQUE ANTERIEURE

Un procédé de mesure de l'indice de cétane de carburants d'alimentation des moteurs diesel et un dispositif pour la mise en oeuvre de ce procédé sont décrits dans le brevet FR 2 701 118.

Selon ce procédé, on mesure la valeur de l'indice de cétane d'un carburant d'alimentation de moteurs diesels par comparaison avec un carburant de référence dont la valeur de l'indice de cétane est connue.

Ces carburants d'alimentation et de référence présentant respectivement des délais d'auto-inflammation, ce procédé consiste à utiliser un moteur diesel de mesure tournant à vitesse constante alimenté successivement par ledit carburant de référence et par ledit carburant d'alimentation, ledit moteur de mesure ayant un taux de compression constant et une avance constante à l'injection et à calculer l'indice de cétane du carburant d'alimentation à partir de la valeur de l'indice de cétane du carburant de référence et à partir des valeurs respectivement mesurées des délais d'auto-inflammation du carburant de référence et du carburant d'alimentation.

L'appareillage connu, utilisé pour mettre en oeuvre ce procédé comprend essentiellement :

- un moteur monocylindre à rapport volumétrique de compression variable,
- une pompe à injection munie d'un moyen micrométrique qui permet de régler manuellement l'avance à l'injection,
- un injecteur de carburant,
- un piston plongeur dont la position est modifiable par action sur un axe de commande, qui permet de régler le taux de compression,
- des moyens de mesure du délai d'auto-inflammation de carburants pouvant alimenter le moteur.

Les résultats obtenus avec cette méthode et cet appareillage sont peu fidèles.

On constate également une dérive importante de la mesure dans le temps, particulièrement gênante lorsque le moteur est utilisé en ligne, pour la mesure en continu de l'indice de cétane d'un carburant pendant sa fabrication.

Ces écarts et cette dérive dans le temps sont dus notamment aux résidus solides de la combustion du carburant appelés calamine, qui se déposent sur les parois de la chambre de combustion du moteur c'est à dire le volume dans lequel s'effectue la compression du mélange air-carburant.

Une méthode connue pour éliminer ces dépôts de calamine consiste à effectuer périodiquement toutes les 50 heures de fonctionnement les opérations suivantes :

- démonter l'injecteur et le piston plongeur,
- arracher mécaniquement la calamine qui s'est déposée sur les parois de ces deux éléments au moyen d'outils appropriés tels que des brosses,
- nettoyer soigneusement ces deux éléments,
- et enfin les remonter.

La durée d'exécution de ces opérations est d'environ une heure, pendant laquelle le moteur est indisponible pour effectuer des mesures. La fréquence et la durée de cette indisponibilité sont un inconvénient pour l'utilisation du moteur en laboratoire. Elle rendent cette méthode inapplicable lorsque le moteur est utilisé pour la mesure en ligne de l'indice de cétane d'un carburant pendant sa fabrication. En effet, les résultats de cette mesure sont utilisés dans un processus de régulation et de contrôle de la qualité du produit fabriqué qui ne supporte pas d'être interrompu pendant une heure.

La fiabilité des résultats obtenus avec cette méthode est médiocre. Par exemple, pour un carburant d'indice de cétane de 49 points on observe des écarts de reproductibilité de l'ordre de 3,8 points.

De plus cette méthode nécessite l'intervention d'un mécanicien qualifié et n'est pas automatisable.

### EXPOSE DE L'INVENTION

La présente invention a justement pour objet de remédier à ces inconvénients et notamment de fournir un procédé et un dispositif automatique de décalaminage d'un moteur de mesure de l'indice de cétane des carburants pour moteurs diesels.

Ce procédé et ce dispositif sont utilisables aussi bien pour les moteurs de laboratoire que pour les moteurs fonctionnant en ligne sur des unités de fabrication de carburants.

A cette fin la présente invention propose un procédé de décalaminage d'une chambre de combustion d'un moteur diesel, moteur destiné à la mesure de l'indice de cétane d'un carburant d'alimentation par comparaison avec l'indice de cétane connu d'un carburant de référence, lesdits carburants étant injectés successivement dans la chambre de combustion préalablement remplie d'air afin de constituer un mélange inflammable, ledit procédé étant caractérisé en ce qu'il consiste à créer entre deux cycles de mesure, pendant 80 à 160 secondes, de fortes turbulences du mélange inflammable et des gaz résultant de sa combustion de manière à décoller au moins partiellement les résidus solides de combustion déposés sur des parois de la chambre de combustion.

Cet effet de décollement résulte de l'augmentation des vitesses de circulation des gaz dans la chambre de combustion, notamment au niveau des parois, par rapport aux vitesses des gaz en régime de fonctionnement stable, qui caractérise les turbulences créées.

Selon une caractéristique préférée du procédé de l'invention, le moteur diesel tournant à une vitesse constante, avec une avance à l'injection prédéterminée et un taux de compression donné, le carburant alimentant le moteur présentant un délai d'auto-inflammation initial, il consiste à exécuter les étapes suivantes :

- diminuer le taux de compression pour obtenir un délai d'auto-inflammation compris entre 1,05 et 1,2 fois le délai d'autoinflammation initial au bout d'un temps compris entre 20 et 40 secondes,
- régler le taux de compression pour maintenir le délai d'auto-inflammation à la valeur atteinte à l'étape précédente, pendant 1 à 10 secondes,
- augmenter le taux de compression pour obtenir un délai d'auto-inflammation compris entre 0,8 et 0,6 fois le délai d'autoinflammation initial au bout d'un temps compris entre 40 et 80 secondes,
- régler le taux de compression pour maintenir le délai d'auto-inflammation à la valeur atteinte à l'étape précédente pendant 1 à 10 secondes,
- diminuer le taux de compression pour ramener le délai d'auto-inflammation à sa valeur initiale dans un temps compris entre 20 et 40 secondes.

La présente invention propose également un dispositif de décalaminage d'une chambre de combustion d'un moteur diesel, moteur destiné à la mesure de l'indice de cétane d'un carburant d'alimentation par comparaison avec l'indice de cétane connu d'un carburant de référence, comportant une chambre de combustion préalablement remplie d'air dans laquelle sont injectés lesdits carburants afin de constituer un mélange inflammable caractérisé en ce qu'il comporte en plus, des moyens pour créer entre deux cycles de mesure, pendant 80 à 160 secondes, de fortes turbulences du mélange inflammable et des gaz résultant de sa combustion, de manière à décoller au moins partiellement les résidus solides de combustion déposés sur des parois de la chambre de combustion.

Selon une caractéristique préférée du dispositif de l'invention, le moteur comportant :

- des moyens de mesure du délai d'auto-inflammation desdits carburants reliés à une sortie d'un capteur de pression,
- un piston plongeur commandable dont la position détermine le taux de compression,

le moteur tournant à vitesse constante, avec une avance à l'injection prédéterminée, avec un taux de compression donné, le carburant alimentant le moteur présentant un délai d'auto-inflammation initial, les moyens pour créer de fortes turbulences du mélange inflammable et des gaz résultant de sa combustion, comportent :

- une unité de traitement reliée à une sortie des moyens de mesure du délai d'auto-inflammation et à des moyens de mémorisation,
- un actionneur relié électriquement à une sortie de l'unité de traitement et mécaniquement à une commande du piston plongeur,

pour faire varier rapidement le délai d'autoinflammation du carburant alimentant le moteur.

Selon une dernière caractéristique préférée du dispositif de l'invention, l'unité de traitement élabore un signal de commande de l'actionneur de manière à exécuter les opérations suivantes :

- modification de la position du piston pour obtenir un délai d'autoinflammation compris entre 1,05 et 1,2 fois le délai d'auto-inflammation initial au bout d'un temps compris entre 20 et 40 secondes,
- réglage de la position du piston pour maintenir le délai d'auto-inflammation à la valeur atteinte à l'issue de l'opération précédente, pendant 1 à 10 secondes,
- modification de la position du piston pour obtenir un délai d'auto-inflammation compris entre 0,8 et 0,6 fois le délai d'auto-inflammation initial au bout d'un temps compris entre 40 et 80 secondes,
- réglage de la position du piston pour maintenir le délai d'auto-inflammation à la valeur atteinte à l'issue de l'opération précédente, pendant 1 à 10 secondes,
- modification de la position du piston pour ramener le délai d'auto-inflammation à sa valeur initiale en un temps compris entre 20 et 40 secondes.

Grâce à l'invention, l'opération de décalaminage rend indisponible le moteur pendant un intervalle de temps relativement court, compris entre 80 et 180 secondes, qui est tout à fait acceptable pour une utilisation d'un moteur dans un laboratoire ou pour la mesure en ligne de l'indice de cétane d'un carburant pendant sa fabrication.

Grâce également à l'invention la fréquence des démontages de l'injecteur et du piston plongeur pour nettoyage peut être diminuée, et par conséquent le nombre des interventions d'un mécanicien spécialiste dans la même proportion, tout en améliorant la fiabilité des résultats des mesures.

Un avantage supplémentaire du procédé de l'invention est son caractère automatisable.

## BREVE DESCRIPTION DES DESSINS

L'invention sera mieux comprise à l'aide de la description suivante, en référence aux dessins annexés dans lesquels :

- la figure 1 représente schématiquement les principaux éléments d'un moteur pour la mesure de l'indice de cétane de carburants pour moteurs diesels et les éléments associés pour son décalaminage conformes au descriptif de l'invention.
- la figure 2 représente l'évolution dans le temps, du délai d'auto-inflammation du carburant alimentant un moteur diesel de mesure en ligne de l'indice cétane d'un carburant conforme au descriptif de l'invention.

## EXPOSE DETAILLE DE L'INVENTION

D'une manière générale, le procédé et le dispositif de l'invention sont utilisés pour décalaminer les moteurs destinés à la mesure de l'indice de cétane des carburants pour moteurs diesels.

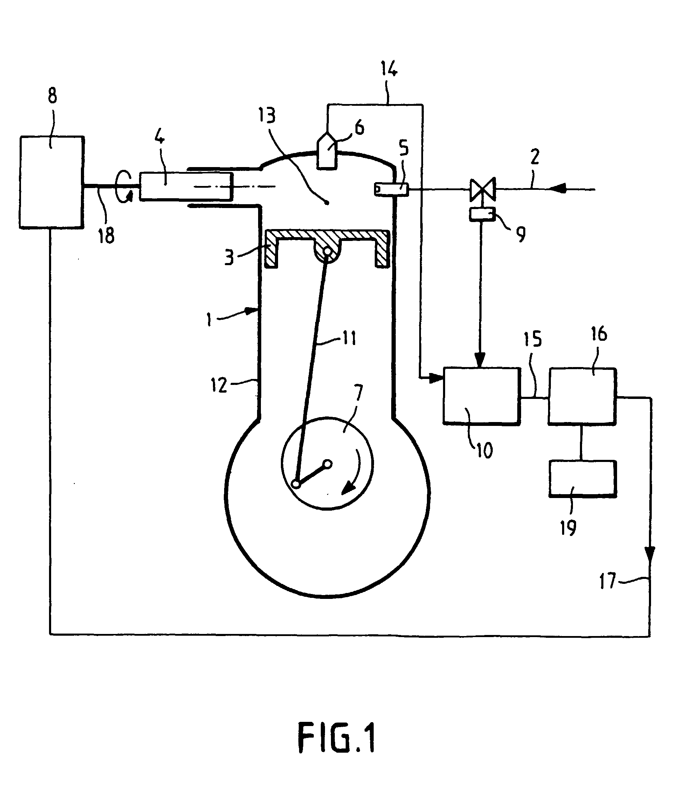

Selon le mode de réalisation représenté sur la figure 1, le dispositif de l'invention comporte :

- un moteur 1 diesel monocylindre, de marque WAUKHESA alimenté en carburant par une canalisation 2 comprenant un cylindre 12, un piston 3, relié à un vilebrequin 7 par une bielle 11, un piston 4 plongeur réglable en position par une commande 18 en rotation, un injecteur 5 de carburant,
- un capteur 9 de détection d'instant d'injection qui fournit une impulsion électrique à l'instant d'injection du carburant dans le moteur,
- un capteur 6 de mesure de la pression dans la chambre 13 de combustion qui fournit sur une sortie 14, un signal électrique représentatif de cette pression,
- des moyens 10 de mesure du délai d'auto-inflammation du carburant alimentant le moteur, reliés à la sortie 14 du capteur 6 et à une sortie du capteur 9.
- un actionneur 8 comportant un moteur à commande pas à pas effectuant 1 tour pour 200 pas de commande, accouplé à un réducteur à engrenages de rapport 1/20, dont l'axe de sortie est relié mécaniquement à la commande 18 en rotation du piston 4 plongeur,
- une unité 16 de traitement reliée à une sortie 15 des moyens 10 de mesure du délai d'auto-inflammation qui délivre sur une sortie 17 un signal de commande à l'actionneur 8 auquel elle est reliée,
- des moyens 19 de mémorisation associés à l'unité 16 de traitement à laquelle ils sont reliés.

Le taux de compression du moteur est réglable par modification de la position du piston 4 plongeur. A chaque tour de la commande 18 en rotation correspond un déplacement longitudinal du piston 4 qui modifie d'une quantité déterminée le volume de la chambre 13 de combustion et par conséquent le taux de compression.

Un cycle de mesure de l'indice de cétane d'un carburant dont on veut déterminer l'indice de cétane se décompose en deux parties, une première partie au cours de laquelle le moteur est alimenté par un carburant de référence d'indice de cétane connu sensiblement égal à 49 et une deuxième partie au cours de laquelle le moteur est alimenté par le carburant dont on veut déterminer la valeur de l'indice de cétane, voisine de celle du carburant de référence.

Ce cycle de mesure se déroule de manière connue de la façon suivante.

Dans la première partie du cycle de mesure le moteur 1 tournant à vitesse constante, par exemple 900 tours par minute, l'avance à l'injection étant maintenue égale à 15° d'angle du vilebrequin, au cours d'une première phase dite de calage le taux de compression est réglé automatiquement pour obtenir un délai d'auto-inflammation du carburant de référence prédéterminé égal à 14° d'angle du vilebrequin.

Ce réglage automatique du taux de compression s'effectue de la manière suivante :

L'unité 16 de traitement, qui reçoit sur une entrée reliée à la sortie 15 des moyens 10 de mesure du délai d'auto-inflammation, un signal électrique représentatif du délai d'auto-inflammation, exécute un premier programme stocké dans les moyens 19 de mémorisation qui compare la valeur de ce signal à une valeur de consigne prédéterminée égale à 14° plus ou moins 0,2° et calcule au moyen d'un algorithme de régulation conventionnel la valeur d'un signal d'action qui est fourni sur la sortie 17.

Ce signal est appliqué à l'entrée de l'actionneur 8 qui modifie la position du piston 4, jusqu'à ce que le délai d'auto-inflammation soit égal à la valeur de consigne prédéterminée.

Au cours d'une deuxième phase de la première partie du cycle de mesure, sans modifier la position du piston plongeur atteinte à la fin de la phase de calage, on mesure la valeur moyenne du délai d'auto-inflammation du carburant de référence pendant environ 55 secondes.

Dans la deuxième partie du cycle de mesure, le moteur étant alimenté par le carburant d'alimentation dont on veut déterminer la valeur de l'indice de cétane, sans modifier le taux de compression du moteur, c'est à dire sans modifier la position atteinte par le piston plongeur à la fin de la phase de calage, au cours d'une première phase on stabilise le fonctionnement du moteur, puis au cours d'une deuxième phase on mesure la valeur moyenne du délai d'auto-inflammation du carburant d'alimentation pendant environ 55 secondes.

L'indice de cétane du carburant d'alimentation est calculé par la formule connue suivante :

$$Icx = Icref + K (Dmes - Dref)$$

dans laquelle :

Icx est la valeur de l'indice de cétane du carburant d'alimentation,

Icref est la valeur de l'indice de cétane du carburant de référence,

Dref est égal à la valeur moyenne du délai d'auto-inflammation du carburant de référence mesurée pendant la deuxième phase de la première partie du cycle de mesure,

Dmes est égal à la valeur moyenne du délai d'auto-inflammation du carburant dont on veut déterminer la valeur de l'indice de cétane, mesurée pendant la deuxième phase de la deuxième partie du cycle de mesure,

K est une constante déterminée expérimentalement par des opérations de calibration préalables du moteur à partir de carburants dont l'indice de cétane est connu avec précision.

Les indices de cétane du carburant de référence et du carburant d'alimentation étant voisins, dans des conditions de fonctionnement identiques du moteur, ces deux carburants présentent des délais d'auto-inflammation voisins.

Le procédé de décalaminage du moteur diesel selon l'invention et mis en oeuvre par le dispositif représenté sur la figure 1 consiste à produire dans la chambre de combustion du moteur des turbulences du mélange inflammable carburant-air et des gaz de combustion qui en résultent.

Ces turbulences sont créées périodiquement tous les trois cycles de mesure, par exemple à la fin de la deuxième partie du troisième cycle de mesure de la manière suivante, le moteur étant alimenté par le carburant de référence. L'unité 16 de traitement exécute un deuxième programme stocké dans les moyens 19 de mémorisation qui à pour effet de déclencher le déroulement des étapes suivantes :

- délivrer sur la sortie 17 de l'unité de traitement un signal électrique de commande de l'actionneur 8 de manière à diminuer le taux de compression pour que le délai d'auto-inflammation du carburant d'alimentation atteigne la valeur de 16° environ, au bout d'un temps égal à 25 secondes environ,

- calculer et délivrer sur la sortie 17 de l'unité de traitement, un signal pour maintenir le délai d'auto-inflammation à cette valeur de 16° pendant environ 5 secondes,

- délivrer sur la sortie 17 de l'unité de traitement, un signal électrique de commande de l'actionneur 8 de manière à augmenter le taux de compression pour que le délai d'auto-inflammation du carburant d'alimentation atteigne la valeur de 10° environ au bout d'un temps égal à 40 secondes environ,

- calculer et délivrer sur la sortie 17 de l'unité de traitement, un signal pour maintenir le délai d'auto-inflammation à cette valeur de 10° pendant environ 5 secondes,

- délivrer sur la sortie 17 de l'unité de traitement, un signal électrique de commande de l'actionneur 8 de manière à diminuer le taux de compression pour que le délai d'auto-inflammation du carburant d'alimentation atteigne la valeur de 14° environ au bout d'un temps égal à 25 secondes environ.

Le déroulement de ces étapes étant terminé une série de trois nouveaux cycles de mesures est enclenchée, à l'issue de laquelle les étapes de décalaminage se déroulent à nouveau.

La reproductibilité des résultats de la mesure de l'indice de cétane obtenue est de 2,5 points avec un démontage et un nettoyage de l'injecteur et du piston plongeur toutes les 100 heures de fonctionnement.

Selon un autre mode de réalisation de l'invention, les étapes de décalaminage se déroulent à la suite de la première partie d'un cycle de mesure au cours de laquelle le moteur est alimenté par un carburant de référence.

Les deux modes de réalisation de l'invention décrits ci-dessus sont donnés à titre d'exemples non limitatifs.

La fréquence des décalaminages sera facilement ajustée expérimentalement en fonction de la nature des carburants, de leurs tendances à encrasser le moteur et de la dérive jugée acceptable de la mesure de l'indice de cétane du carburant d'alimentation pour une application donnée.

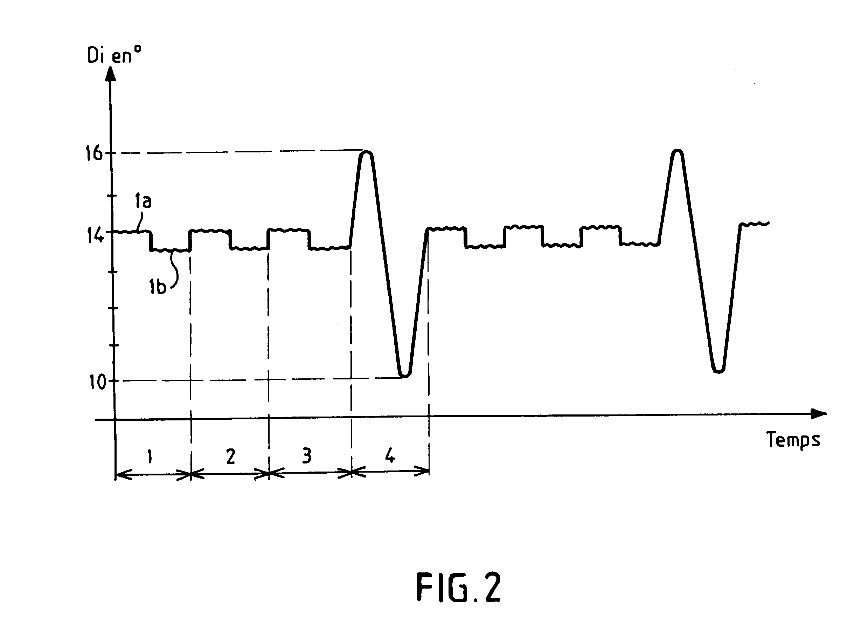

La figure 2 représente l'évolution du délai Di d'auto-inflammation du carburant alimentant le moteur, exprimé en degrés d'angle de rotation du vilebrequin en fonction du temps conformément au premier mode de réalisation précédemment décrit.

Les intervalles de temps 1, 2 et 3 correspondent à trois cycles de mesure successifs, chacun d'eux comportant une première partie au cours de laquelle le moteur est alimenté par le carburant de référence d'indice de cétane connu et une deuxième partie au cours de laquelle le moteur est alimenté par le carburant d'alimentation dont on veut déterminer l'indice de cétane. Pour le cycle de mesure qui se déroule pendant l'intervalle de temps 1, ces deux parties de cycle de mesure sont respectivement repérées 1a et 1b.

Les étapes du procédé de décalaminage de l'invention se déroulent pendant l'intervalle de temps 4 au cours duquel on constate clairement les fluctuations du délai d'auto-inflammation du carburant alimentant le moteur provoquées par les variations du taux de compression du moteur, conformément au procédé de l'invention.

**Revendications**

1. Procédé de décalaminage d'une chambre de combustion d'un moteur diesel (1), moteur destiné à la mesure de l'indice de cétane d'un carburant d'alimentation par comparaison avec l'indice de cétane connu d'un carburant de référence, lesdits carburants étant injectés successivement dans la chambre (13) de combustion préalablement remplie d'air afin de constituer un mélange inflammable, ledit procédé étant caractérisé en ce qu'il consiste à créer entre deux cycles de mesure, pendant 80 à 160 secondes, de fortes turbulences du mélange inflammable et des gaz résultant de sa combustion de manière à décoller au moins partiellement les résidus solides de combustion déposés sur des parois de la chambre (13) de combustion.

2. Procédé selon la revendication 1 caractérisé en ce que le moteur diesel (1) tournant à une vitesse constante, avec une avance à l'injection prédéterminée et un taux de compression donné, le carburant alimentant le moteur présentant un délai d'auto-inflammation initial, il consiste à exécuter les étapes suivantes :

   - diminuer le taux de compression pour obtenir un délai d'auto-inflammation compris entre 1,05 et 1,2 fois le délai d'autoinflammation initial au bout d'un temps compris entre 20 et 40 secondes,
   - régler le taux de compression pour maintenir le délai d'auto-inflammation à la valeur atteinte à l'étape précédente, pendant 1 à 10 secondes,
   - augmenter le taux de compression pour obtenir un délai d'auto-inflammation compris entre 0,8 et 0,6 fois le délai d'autoinflammation initial au bout d'un temps compris entre 40 et 80 secondes,
   - régler le taux de compression pour maintenir le délai d'auto-inflammation à la valeur atteinte à l'étape précédente pendant 1 à 10 secondes,
   - diminuer le taux de compression pour ramener le délai d'auto-inflammation à sa valeur initiale dans un temps compris entre 20 et 40 secondes.

3. Dispositif de décalaminage d'une chambre (13) de combustion d'un moteur diesel (1), moteur destiné à la mesure de l'indice de cétane d'un carburant d'alimentation par comparaison avec l'indice de cétane connu d'un carburant de référence, comportant une chambre (13) de combustion préalablement remplie d'air dans laquelle sont injectés lesdits carburants afin de constituer un mélange inflammable caractérisé en ce qu'il comporte en plus, des moyens pour créer entre deux cycles de mesure, pendant 80 à 160 secondes, de fortes turbulences du mélange inflammable et des gaz résultant de sa combustion, de manière à décoller au moins partiellement les résidus solides de combustion déposés sur des parois de la chambre (13) de combustion.

4. Dispositif selon la revendication 3 caractérisé en ce que le moteur (1) comportant :

   - des moyens (10) de mesure du délai d'auto-inflammation desdits carburants reliés à une sortie (14) d'un capteur (6) de pression dans la chambre de combustion et à un capteur (9) de détection de l'instant d'injection,
   - un piston (4) plongeur commandable dont la position détermine le taux de compression du moteur (1),

   le moteur (1) tournant à vitesse constante, avec une avance à l'injection prédéterminée, avec un taux de compression donné, le carburant alimentant le moteur présentant un délai d'auto-inflammation initial, les moyens pour créer de fortes turbulences du mélange inflammable et des gaz résultant de sa combustion, comportent :

   - une unité (16) de traitement reliée à une sortie (15) des moyens (10) de mesure du délai d'auto-inflammation et à des moyens (19) de mémorisation,
   - un actionneur (8) relié électriquement à une sortie (17) de l'unité de traitement et mécaniquement à une commande (8) du piston (4) plongeur,

   pour faire varier rapidement le délai d'auto-inflammation du carburant alimentant le moteur.

5. Dispositif selon la revendication 4 caractérisé en ce que l'unité (16) de traitement élabore un signal de commande de l'actionneur (8) de manière à exécuter les opérations suivantes :

   - modification de la position du piston (4) pour obtenir un délai d'autoinflammation compris entre 1,05 et 1,2 fois le délai d'auto-inflammation initial au bout d'un temps compris entre 20 et 40 secondes,
   - réglage de la position du piston (4) pour maintenir le délai d'auto-inflammation à la valeur atteinte à l'issue de l'opération précédente, pendant 1 à 10 secondes,
   - modification de la position du piston (4) pour obtenir un délai d'auto-inflammation compris entre 0,8 et 0,6 fois le délai d'auto-inflammation initial au bout d'un temps compris entre 40 et 80 secondes,
   - réglage de la position du piston (4) pour maintenir le délai d'auto-inflammation à la valeur atteinte à l'issue de l'opération précédente, pendant 1 à 10 secondes,

- modification de la position du piston (4) pour ramener le délai d'auto-inflammation à sa valeur initiale en un temps compris entre 20 et 40 secondes.

**Patentansprüche**

1. Verfahren zur Ölkohleentfernung aus einer Verbrennungskammer eines Dieselmotors (1), der zur Messung der Cetanzahl eines Speisungskraftstoffs im Vergleich zur bekannten Cetanzahl eines Bezugskraftstoffs bestimmt ist, wobei die Kraftstoffe nacheinander in die Verbrennungskammer (13) eingespritzt werden, die vorher mit Luft gefüllt wurde, um ein brennbares Gemisch zu bilden, dadurch **gekennzeichnet**, dass das Verfahren darin besteht, dass man zwischen zwei Messzyklen während 80 bis 160 Sekunden starke Turbulenzen des brennbaren Gemisches und sich aus seiner Verbrennung ergebender Gase erzeugt, um wenigstens teilweise die auf den Wandungen der Verbrennungskammer (13) niedergeschlagenen festen Verbrennungsrückstände zu entfernen.

2. Verfahren nach Anspruch 1, dadurch **gekennzeichnet**, dass bei konstanter Drehzahl des Dieselmotors, bei vorgegebener Voreinspritzung, einem gegebenen Verdichtungsverhältnis und einem anfänglichen Selbstzündungsverzug des den Motor speisenden Kraftstoffs es in der Ausführung folgender Schritte besteht:

- Verringerung des Verdichtungsverhältnisses zur Erzielung eines 1,05- bis 1,2-fachen Selbstzündungsverzugs bezogen auf den anfänglichen Selbstzündungsverzug nach einer Zeitdauer zwischen 20 und 40 Sekunden,
- Einstellung des Verdichtungsverhältnisses zur Aufrechterhaltung des Selbstzündungsverzugs bei dem bei dem vorhergehenden Schritt erzielten Wert während 1 bis 10 Sekunden,
- Steigerung des Verdichtungsverhältnisses zur Erzielung eines 0,8- bis 0,6-fachen Selbstzündungsverzugs bezogen auf den anfänglichen Selbstzündungsverzug nach einer Zeitdauer zwischen 40 und 80 Sekunden,
- Einstellung des Verdichtungsverhältnisses zur Aufrechterhaltung des Selbstzündungsverzugs bei dem bei dem vorhergehenden Schritt erzielten Wert während 1 bis 10 Sekunden,
- Verringerung des Verdichtungsverhältnisses zur Rückführung des Selbstzündungsverzugs auf den Anfangswert in einer Zeit zwischen 20 und 40 Sekunden.

3. Vorrichtung zur Ölkohleentfernung aus einer Verbrennungskammer eines Dieselmotors (1), der zur Messung der Cetanzahl eines Speisungskraftstoffs im Vergleich zur bekannten Cetanzahl eines Bezugkraftstoffs bestimmt ist, mit einer vorher mit Luft gefüllten Verbrennungskammer (13), in die die Kraftstoffe eingespritzt werden, um ein brennbares Gemisch zu bilden, dadurch **gekennzeichnet**, dass sie außerdem noch Einrichtungen aufweist, die zwischen zwei Messzyklen während 80 bis 160 Sekunden starke Turbulenzen des brennbaren Gemischs und sich aus seiner Verbrennung ergebender Gase erzeugen, um wenigstens teilweise die auf den Wandungen der Verbrennungskammer (13) niedergeschlagenen festen Verbrennungsrückstände zu entfernen.

4. Vorrichtung nach Anspruch 3, dadurch **gekennzeichnet**, dass der Motor (1)

- Einrichtungen (10) zur Messung des Selbstzündungsverzugs der Kraftstoffe, die mit einem Ausgang (14) eines Druckfühlers (6) in der Verbrennungskammer und mit einem Fühler (9) zur Ermittlung des Zeitpunkts der Einspritzung verbunden sind, und
- einen steuerbaren Tauchkolben (4) aufweist, dessen Stellung das Verdichtungsverhältnis des Motors (1) bestimmt,

wobei der Motor (1) bei vorgegebener Voreinspritzung, einem gegebenen Verdichtungsverhältnis und einem anfänglichen Selbstzündungsverzug des den Motor speisenden Kraftstoffs mit konstanter Drehzahl dreht, und wobei die Einrichtungen zur Erzeugung von starken Turbulenzen des brennbaren Gemisches und sich aus seiner Verbrennung ergebender Gase

- eine mit einem Ausgang (15) der Einrichtungen (10) zur Messung des Selbstzündungsverzugs und mit Speichereinrichtungen (19) verbundene Verarbeitungseinheit (16) und
- ein mit einem Ausgang (17) der Verarbeitungseinheit elektrisch und mit einem Antrieb (8) des Tauchkolbens (4) mechanisch verbundenes Stellglied (8) aufweisen, um den Selbstzündungsverzug des den Motor speisenden Kraftstoffs rasch ändern zu können.

5. Vorrichtung nach Anspruch 4, dadurch **gekennzeichnet**, dass die Verarbeitungseinheit (16) ein Steuersignal des Stellgliedes (8) erzeugt, um die folgenden Vorgänge auszuführen:

- Änderung der Stellung des Kolbens (4) zur Erzielung eines 1,05- bis 1,2-fachen Selbstzündungsverzugs bezogen auf den anfänglichen Selbstzündungsverzug nach einer Zeitdauer zwischen 20 und 40 Sekunden,

- Einstellung der Stellung des Kolbens (4) zur Aufrechterhaltung des Selbstzündungsverzugs bei dem als Ergebnis des vorhergehenden Vorgangs erzielten Wert während 1 bis 10 Sekunden,

- Änderung der Stellung des Kolbens (4) zur Erzielung eines 0,8- bis 0,6-fachen Selbstzündungsverzugs bezogen auf den anfänglichen Selbstzündungsverzug nach einer Zeitdauer zwischen 40 und 80 Sekunden,

- Einstellung der Stellung des Kolbens (4) zur Aufrechterhaltung des Selbstzündungsverzugs bei dem als Ergebnis des vorhergehenden Vorgangs erzielten Wert während 1 bis 10 Sekunden und

- Änderung der Stellung des Kolbens (4) zur Rückführung des Selbstzündungsverzugs auf den Anfangswert in einer Zeit - zwischen 20 und 40 Sekunden.

**Claims**

1. Process for decarbonising a combustion chamber of a diesel engine (1), which engine is intended to measure the cetane index of a supplied motor fuel in comparison with the known cetane index of a reference motor fuel, the motor fuels being injected successively into the combustion chamber (13) previously filled with air in order to constitute an inflammable mixture, the process being characterised in that it consists in creating, between two measurement cycles, for from 80 to 160 seconds, violent turbulence of the inflammable mixture and of the gases resulting from the combustion thereof in order to loosen at least partially the solid combustion residues deposited on walls of the combustion chamber (13).

2. Process according to claim 1, characterised in that, with the diesel engine (1) turning at a constant speed, with a predetermined injection advance and a given compression ratio, and the motor fuel supplying the engine having an initial self-ignition delay, the following steps are to be carried out:

- reduction in the compression ratio in order to obtain a self-ignition delay of between 1.05 and 1.2 times the initial self-ignition delay at the end of a period of between 20 and 40 seconds,

- regulation of the compression ratio in order to maintain the self-ignition delay at the value obtained in the preceding step for from 1 to 10 seconds,

- increase in the compression ratio in order to obtain a self-ignition delay of between 0.8 and 0.6 times the initial self-ignition delay at the end of a period of between 40 and 80 seconds,

- regulation of the compression ratio in order to maintain the self-ignition delay at the value obtained in the preceding step for from 1 to 10 seconds,

- reduction in the compression ratio in order to return the self-ignition delay to its initial value within a period of between 20 and 40 seconds.

3. Device for decarbonising a combustion chamber (13) of a diesel engine (1), which engine is intended to measure the cetane index of a supplied motor fuel in comparison with the known cetane index of a reference motor fuel, comprising a combustion chamber (13) previously filled with air, into which are injected the motor fuels in order to constitute an inflammable mixture, characterised in that it further comprises means for creating, between two measurement cycles, for from 80 to 160 seconds, violent turbulence of the inflammable mixture and of the gases resulting from the combustion thereof in order to loosen at least partially the solid combustion residues deposited on walls of the combustion chamber (13).

4. Device according to claim 3, characterised in that, with the engine (1) comprising:

- means (10) for measuring the self-ignition delay of the motor fuels, connected to an outlet (14) of a pressure sensor (6) in the combustion chamber and to a sensor (9) for detecting the moment of injection,

- a controllable plunger piston (4) whose position determines the compression ratio of the engine (1),

the engine (1) turning at a constant speed, with a predetermined injection advance, with a given compression ratio, and the motor fuel supplying the engine having an initial self-ignition delay, the means for creating violent turbulence of the inflammable mixture and of the gases resulting from the combustion thereof comprise:

- a processing unit (16) which is connected to an outlet (15) for means (10) for measuring the self-ignition delay and to storage means (19),

- an activating element (8) connected electrically to an outlet (17) of the processing unit and mechanically to a control (18) of the plunger piston (4),

in order rapidly to vary the self-ignition delay of the motor fuel supplying the engine.

5. Device according to claim 4, characterised in that

**EP 0 895 080 B1**

the processing unit (16) processes a control signal from the activating element (8) in order to execute the following operations:

- modification of the position of the piston (4) in order to obtain a self-ignition delay of between 1.05 and 1.2 times the initial self-ignition delay at the end of a period of between 20 and 40 seconds,
- regulation of the position of the piston (4) in order to maintain the self-ignition delay at the value obtained at the end of the preceding operation for from 1 to 10 seconds,
- modification of the position of the piston (4) in order to obtain a self-ignition delay of between 0.8 and 0.6 times the initial self-ignition delay at the end of a period of between 40 and 80 seconds,
- regulation of the position of the piston (4) in order to maintain the self-ignition delay at the value obtained at the end of the preceding operation for from 1 to 10 seconds,
- modification of the position of the piston (4) in order to return the self-ignition delay to its initial value within a period of between 20 and 40 seconds.

FIG.1

FIG.2

EP 0 895 080 B1